# EUROPEAN PATENT SPECIFICATION

(11) **EP 0 454 813 B1**
(45) Date of publication and mention of the grant of the patent: **09.09.1998**
(21) Application number: 90916537.5
(22) Date of filing: 30.10.1990
(51) Int. Cl.: A61K 38/17

(54) **A CASEIN FRACTION FOR THERAPEUTIC, PROPHYLACTIC AND/OR DIAGNOSTIC USE IN INFECTIONS OF THE RESPIRATORY TRACT**
EINE CASEINFRAKTION ZUR THERAPEUTISCHEN VORBEUGENDEN UND/ODER DIAGNOSTISCHEN VERWENDUNG BEI ATEMWEGINFEKTIONEN
FRACTION DE CASEINE A USAGE THERAPEUTIQUE, PROPHYLACTIQUE ET/OU DIAGNOSTIQUE DANS DES INFECTIONS DES VOIES RESPIRATOIRES

(30) Priority: 30.10.1989 SE 8903625
(43) Date of publication of application: 06.11.1991
(73) Proprietor: ANDERSSON, Bengt, S-431 34 Mölndal (SE); ANIANSSON, Gustaf, S-211 49 Malmö (SE); LINDSTEDT, Ragnar, S-223 59 Lund (SE); SVANBORG EDEN, Catharina, S-223 59 Lund (SE)
(72) Inventor: ANDERSSON, Bengt, S-431 34 Mölndal (SE); ANIANSSON, Gustaf, S-211 49 Malmö (SE); LINDSTEDT, Ragnar, S-223 59 Lund (SE); SVANBORG EDEN, Catharina, S-223 59 Lund (SE)
(74) Representative: Greaves, Carol Pauline
(86) International application number: SE9000702
(87) International publication number: WO9106308

(56) References cited:
- EP-A- 0 049 666
- EP-A- 0 291 265
- FR-A- 2 592 769
- GB-A- 1 496 671
- Dialog Information Services, File 155, Medline 67-91, NLM accessionnumber 91014641, ANIANSSON G et al: "Anti-adhesive activity of human casein against Streptococcus pneumoniae and Haemophilus influenzae" Microb Pathog May 1990, 8 (5) p 315-23.
- Dialog Information Services, File 155, Medline 67-91, NLM accessionnumber 89341079, MIGLIORE-SAMOUR D et al: "Biologically active casein peptides implicated in immunomodulation", J Dairy Res 1989, 56 (3) p 357-62.

## Description

### Technical field

The present invention relates to novel antibacterial compositions, in the form of pharmaceutical compositions, human food compositions, and animal feedstuffs to be used in the therapeutic and/or prophylactic treatment of infections caused by Streptococcus pneumoniae and/or Heamophilus influenzae, as well as a method for diagnosing infections caused by said bacteria.

The object of the present invention is to obtain compositions for prophylactic and/or therapeutic treatment of infections caused by Streptococcus pneumoniae and Haemophilus influenzae in the upper airways, ear-nose-and-throat infections, but also in the lower airways, e.g., the lungs by preventing adhesion of these bacteria, and/or killing the same. A further object is to be able to diagnose infections caused by these bacteria.

### Background of the invention

Glycoconjugates interact specifically with microbial lectins, e.g. viral envelope proteins, bacterial adhesins and toxins (20). The specificity is provided by saccharide sequences, e.g. the GM1 ganglioside recognized by cholera toxin (9,10), Gal 1 -- 4Gal by uropathogenic E. coli (14,16), or GlcNAc 1 --3Gal by Streptococcus pneumoniae (2). When membrane bound, the glycoconjugates act as receptors, and the interaction with e.g. bacteria results in the attachment thereof to the receptor-bearing cell (17). When secreted these oligosaccharide sequences have other functions. For example, the presence in external secretions of oligosaccharide sequences corresponding to the cell-bound receptors provides the basis for competitive inhibition of microbial binding (3). Since attachment to epithelial cells is an important event in the pathogenesis of many bacterial infections, inhibition of attachment by secreted oligosaccharides may protect against infection. (EP-A1-0 126 043).

Human milk is a rich source of free oligosaccharides and glycoconjugates (12,13). Human milk inhibits the attachment both of S pneumoniae and H influenzae nasopharyngeal epithelial cells in vitro (3). Inhibitors for pneumococcal binding were identified in the free oligosaccharide fraction, as expected from its content of lacto-and neolactotetraose with known receptor activity for pneumococci (3).

Additional components of a molar weight of > 5000 dalton but not of immunoglobulin nature were also to interact with both S. pneumoniae and H. influenzae (3). The present invention describes the identification of these non-immunoglobulin components in the high molecular weight fraction of human milk as caseins.

EP-A-0 291 265 relates to a sialic acid conjugated protein from bovine milk which is disclosed having infection protecting properties. The protein part is a glycoprotein, and a kappacasien, lactoferrin. The protectant is disclosed having adhesion inhibitory effects on E. coli, and a hemagglutination inhibitory effect on influenza A (Nigata strain) and influenza B (Singapore strain).

### Description of the present invention

It has now surprisingly been found possible to improve the antibacterial effect against infections caused by S. pneumoniae, and/or H influenzae by administering, prophylactically or therapeutically, a composition comprising a casein fraction where the active ingredient is not alfa or beta casein, derived from human milk and having a molecular weight of at least 5000 dalton besides commonly known inert acceptable fillers and expedients or nutrients.

Further characteristics of the invention will be evident from the accompanying claims.

The present invention will be described more in detail with reference to the example given below.

The term milk used in the following will mean either mature or colostrum milk, unless otherwise stated. The milks used is of human origin.

### EXPERIMENTAL

### Bacteria

S. pneumoniae (CCUG3114) and H influenzae (Hi198) were used throughout the experiments. These strains were initially isolated from the nasopharynx of children with frequent episodes of acute otitis media. CCUG3114 (capsular type 6A) was used previously as a model strain in the studies defining the oligosaccharide binding and receptor specificity of pneumococci (2). The strains were kept lyophilized. The lyophils were transferred to blood agar (CCUG3114) or Levinthal medium agar plates (Hi198) (18). CCUG3114 was cultured for 10 hrs at 37°C in liquid medium (15), harvested by centrifugation at 1500 g for 15 min. and suspended in 1 ml of 0.85% NaCl with 1% choline. H. influenzae Hi198 was cultured for 4 hrs in haemophilus medium (5), harvested by centrifugation and suspended in phosphate-buffer saline, PBS, (Hi198).

### Milk

A pool of human milk from healthy donor mothers was used when outdated for nutritional purposes. The mature milk were centrifuged at 2500xg for 15 min, and the fat was removed.

### Adhesion inhibition

Adhesion was tested as previously described (1, 22). In brief, epithelial cells from the oropharynx of healthy donors (10⁵/ml) were mixed with the bacterial suspensions (10⁹/ml). After centrifugation at 400xg and incubation of bacteria and epithelial cells, unbound bacteria were eliminated by repeated cycles of centrifugation at 100xg and resuspension in NaCl with 1% choline.

The inhibitory activity of milk and milk components was tested by preincubation of bacteria for 30 min at 37°C. Epithelial cells were subsequently added and adherence testing continued as described. The number of bacteria attached to the epithelial cells was counted by interference contrast microscopy (Ortolux II microscope with interference contrast equipment TE Leitz, Wetzlar, FRG). Adherence was given as the mean number of bacteria/cell for 40 epithelial cells. Inhibition was given in per cent of the value of the buffer control. EID₅₀ = the effective inhibitory dose required for 50% inhibition of attachment.

### Statistical analysis

The differences in adherence between the saline control and the milk samples were evaluated using the chi-square test for the medium. The inhibitory effect of the milk components was defined by pairwise test of means of repeated experiments.

The attachment to human nasopharyngeal epithelial cells of S. pneumoniae and H. influenzae was inhibited by mature human but not by mature bovine milk (Table 1).

**TABLE 1**

| Adhesion inhibition by human and bovine milk | | | | | | |
|---|---|---|---|---|---|---|
| Inhibitor | Adhesion | | | | | |
| | Bacteria/cell (% of saline control) | | | | | |
| | Pneumococci | | | H, influenzae | | |
| | Mean | % | p< | Mean | % | p< |
| Saline control | 51 | 100 | | 98 | 100 | |
| Mature milk, bovine | 55 | 108 | ns | 86 | 88 | ns |
| Mature milk, human | 4 | 8 | 0.001 | 29 | 30 | 0.001 |
| HMWF, human | 6 | 12 | 0.001 | 42 | 43 | 0.01 |
| LMWF, human | 31 | 61 | 0.01 | 92 | 94 | ns |
| Mean values based upon two experiments. p = compared to the saline control. | | | | | | |

The nature of the inhibitory components in human milk was first analysed by separation according to molecular weight. The whole milk and the whey fractions were separated into fractions comprising high and low molecular weight components by passage over a G-25 Sephadex^{R} column (cut-off 5000 dalton molecular weight, PD10, Pharmacia AB, Uppsala, Sweden) the high molecular weight fraction (HMWF) (in 3.5 ml distilled water) was treated directly. As expected from previous studies inhibitory activity for S. pneumoniae was found in the fraction <5000 dalton containing free oligosaccharides. The LMWF did, however, not influence the attachment of H. influenzae (Table 1).

### Casein

Casein was isolated by the method of, e.g., Mellander (19). To 0.5 l of fat-free human milk 13.5 ml of 10% potassium oxalate was added. After over night incubation at +4°C the precipitate was removed by centrifugation at 2500xg for 15 min. Distilled water was added and 0.5 mmol HCl was slowly stirred into the suspension to a final pH of 4.6. The solution was heated to 30°C for 1 hr and subsequently left over night at +4°C. After centrifugation 2500xg for 15 min the supernatant was discarded and the precipitate was washed by 3-5 cycles of resuspension in distilled water and centrifugation. The preparation was used fresh or kept lyophilized.

The milk was further separated into the casein and whey fractions. At a concentration corresponding to that in milk (2 mg/ml) the casein fraction (HMWF) inhibited the attachment both of S. pneumoniae and H. influenzae (Table 2).

**TABLE 2**

| Adhesion inhibition by the casein fraction of human milk. | | | | | | |
|---|---|---|---|---|---|---|
| Inhibitor | Adhesion | | | | | |
| | Bacteria/cell (% of saline control) | | | | | |
| | Pneumococci | | | H. influenzae | | |
| | Mean | % | p< | Mean | % | p< |
| Saline control | 106 | 100 | | 110 | 100 | |
| Human milk | 4 | 4 | 0.001 | 35 | 38 | 0.001 |
| Whey fraction | 44 | 44 | 0.01 | 101 | 92 | ns |
| Casein fraction | 13 | 12 | 0.001 | 31 | 28 | 0.001 |
| Bovine milk | 100 | 91 | ns | 100 | 91 | ns |
| Whey fraction | 115 | 108 | ns | 120 | 109 | ns |
| Casein fraction | 131 | 124 | ns | 90 | 85 | ns |
| Mean values based upon two experiments. p = compared to the saline control. | | | | | | |

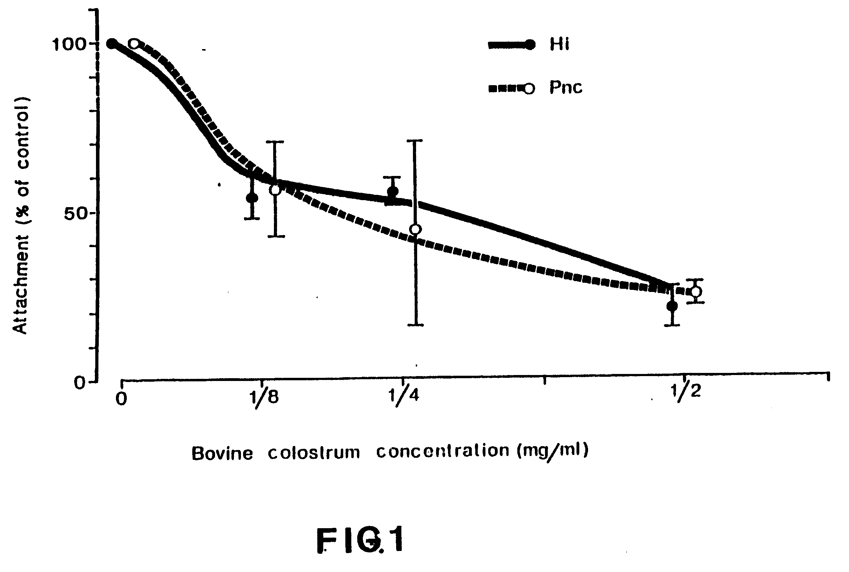

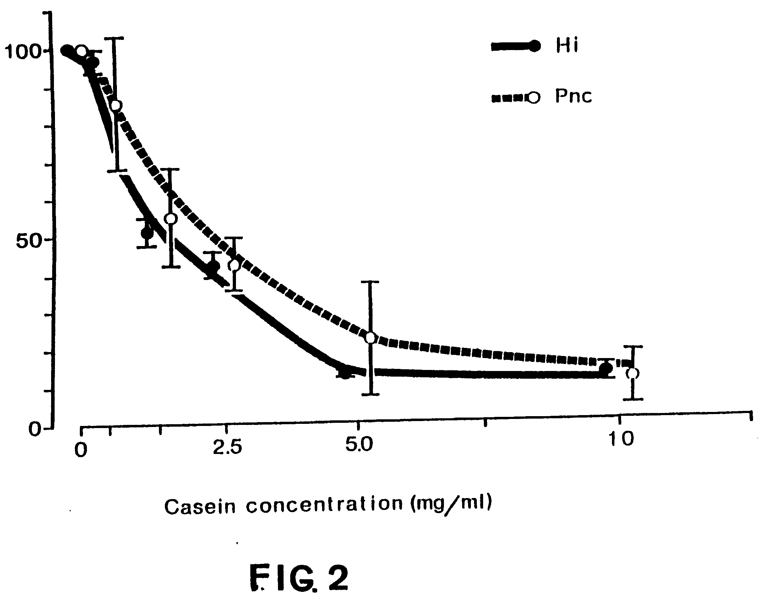

The inhibitory activity of the case in fraction was confirmed with purified, commercially available human casein. On a weight basis the casein fraction and the commercial fraction were equally active. The relationship between the casein concentration and the degree of adhesion inhibition is shown in FIG. 2. At 10 mg/ml the casein fraction reduced pneumococcal attachment by more than 80%. The EID₅₀ was about 2 mg/ml. For H. influenzae a similar dose response curve was obtained with a maximal inhibition of 86% at 10 mg/ml and an EID₅₀ value of 2 mg/ml.

The species difference in inhibitory activity between mature human milk and bovine milk was confirmed for the casein and whey fractions of bovine milk, neither of which inhibited the attachment of S. pneumoniae and H. influenzae (Table 2).

The inhibitory activity for H. influenzae was restricted to the casein fraction of human milk; no activity remained in the whey fraction (Table 2). In contrast pneumococcal attachment was inhibited also by the whey fraction. The molecular size of the inhibitors of the whey fraction was analysed after separation into components above and below 5000 daltons. Both the high and the low molecular weight fractions possessed inhibitory activity (Table 3).

Purified commercial components of human milk; albumin, casein, alfa-lactalbumin, and lactoferrin were purchased from Sigma Chemical Company, St. Louis, Mo, USA.

The commercially available whey proteins of human and bovine origin had no inhibitory activity for S. pneumoniae (Table 3) or H. influenzae.

**TABLE 3**

| Components of whey and anti-adhesive activity for S. pneumoniae | | | |
|---|---|---|---|
| Inhibitor | Adhesion | | |
| | Bacteria/cell (% of saline control | | |
| | Mean | % | p< |
| Fractions of human milk | | | |
| Saline control | 48 | 100 | |
| Whey, total | 15 | 31 | 0.001 |
| Whey, HMWF | 20 | 42 | 0.01 |
| Whey, LMWF | 26 | 54 | 0.01 |
| Whey, boiled | 22 | 46 | 0.01 |

| Commercial whey proteins | | | |
|---|---|---|---|
| Saline control | 72 | 100 | |
| Human albumin | 68 | 85 | ns |
| Human alfa-lactalbumin | 95 | 132 | ns |
| Human lactoferrin | 104 | 144 | ns |
| Bovine alfa-lactalbumin | 131 | 185 | ns |
| Bovine lactoferrin | 59 | 82 | ns |
| Mean values based upon two experiments. p = compared to saline control. The concentration of whey components was 1/2 of that in milk. The concentration of commercial whey proteins was 20 mg/ml. | | | |

A fraction rich in kappa-casein glycopeptide was obtained by the method of Jollés et al as follows: chymosin (Sigma; enzyme:substrate 1:2000) was added to a 2% solution of whole casein in a 0.2 M pyridin:acetic acid buffer at pH 5.5. After digestion during 1.5 hrs at 37°C trichloroacetic acid (final concentration 12%) was added and gently mixed for 16 hrs at +4°C. The supernatant obtained after centrifugation at 14300xg for 2 hrs at +4°C contained the kappa-casein glycopeptide. The latter was washed six times using diethyl ether, dialyzed against distilled water at 4°C and lyophilized.

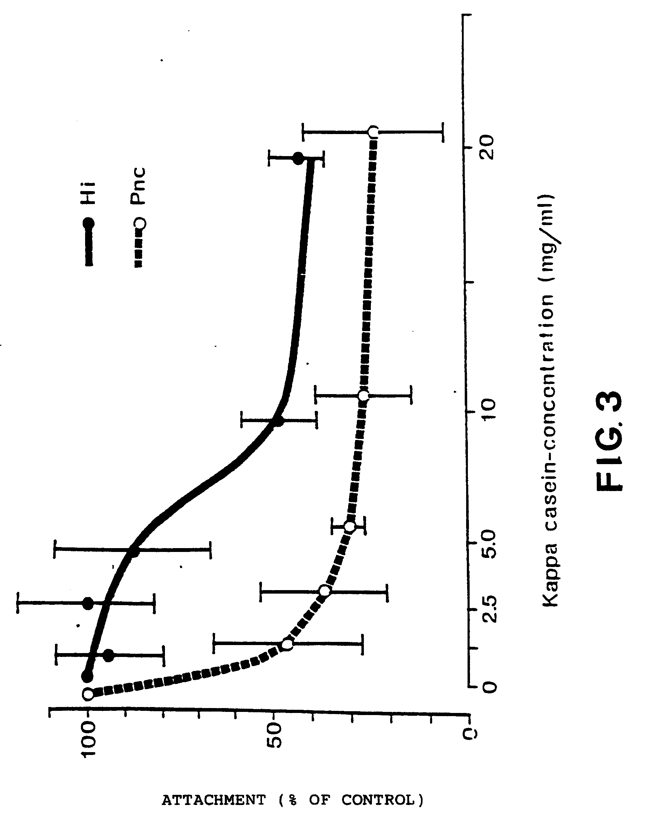

The casein fraction consisting mainly of kappa-casein was found to inhibit the attachment of both S. pneumoniae and H. influenzae (FIG. 3). For S. pneumoniae the EID₅₀ was about 2 mg/ml, i.e., comparable to the unseparated casein fraction. In contrast the inhibitory activity against H. influenzae was significantly reduced to an EID₅₀ of about 10 mg/ml. At a concentration of 10 mg/ml the residual (alfa and beta casein) had no inhibitory activity.

### Bactericidal activity of casein

The bactericidal activity of the casein fraction was tested as follows: 1x10⁹ bacteria/ml determined in a haemocytometer and casein fraction were mixed and incubated at 37°C for 30 minutes. After dilution to concentrations between 1x10² and 1x10⁸ bacteria/l the bacteria were grown on agar plates over night. The number of bacterial colonies on the plates were counted and compared with bacteria incubated with saline buffer.

The amount of viable bacteria of S. pneumoniae (CCUG 3114) after treatment with the human milk casein fraction (2 mg/ml) was determined as well as the amount of viable bacteria of H. influenzae (Hi 198) and K88 positive E. coli. The results obtained are given in Table 4 below.

**TABLE 4**

| Species | Treatment | Viable counts | | | |
|---|---|---|---|---|---|
| | | 0' | 10' | 30' | 120' |
| S. peumoniae | saline | 4,4x10⁸ | 4,0x10⁸ | 2,6x10⁸ | 4,0x10⁷ |
| | casein | 3,0x10⁸ | 5,2x10⁸ | <1,0x10² | <1,0x10² |
| H.influenzae | saline | 1,0x10⁹ | 8,0x10⁸ | 1,0x10⁹ | 2,0x10⁸ |
| | casein | 2,0x10⁸ | 2,0x10⁸ | 2,0x10⁸ | 1,0x10⁸ |
| E.coli K88 | saline | 1,0x10⁸ | 3,0x10⁸ | 5,4x10⁸ | 7,0x10⁸ |
| | casein | 5,0x10⁸ | 4,0x10⁸ | 1,2x10⁹ | 1,0x10⁸ |

As evident from Table 4 above the number of living bacteria was considerably reduced when human milk casein was used, which indicates that human milk casein not only inhibits adhesion, but also possesses an bacteriocidal effect.

The adherence inhibition and the bacteriocidal effect were found to be independent from each other. The adhesive activity of S. pneumoniae and H. influenzae was retained after formalin fixation and casein inhibited attachment also of fixed bacteria. Test results are shown in Table 5 below.

**TABLE 5**

| Casein Preparation | Adhesion Bacteria/cell | | Viability Viable units | |
|---|---|---|---|---|
| | Untreated | Formalin | Untreated | Formalin |
| S. peumoniae | | | | |
| saline | 30 | 27 | 1,3x10⁸ | 0 |
| casein | 1 | 11 | <1,0x10² | 0 |

| H.influenzae | | | | |
|---|---|---|---|---|
| saline | 43 | 44 | 3,0x10⁹ | 0 |
| casein | 19 | 5 | 2,0x10⁸ | 0 |

The bacteriocidal and anti-adhesive components of casein were separated in the organic and aqueous phase. The results obtained as to adhesion inhibition and viability are given in Table 6 below.

**TABLE 6**

| | % inhib. Bact/cell | Incub. of control | Time | Viable counts |
|---|---|---|---|---|
| S.pneumoniae | | | | |
| NaCl (control) | 51 | 100 | 30 | 1,3x10⁹ |
| Casein 2 mg/ml | 18 | 35 | 30 | 1,0x10³ |
| Casein organic phase | 5 | 9 | 30 | 4,0x10³ |
| Casein water phase | 28 | 54 | 30 | 1,2x10⁹ |

| H.influenzae | | | | |
|---|---|---|---|---|
| PBS (control) | 48 | 100 | 30 | 3,0x10⁹ |
| Casein 2 mg/ml | 17 | 35 | 30 | 2,2x109 |
| Casein organic phase | 49 | 102 | 30 | 4,0x10⁹ |
| Casein water phase | 16 | 33 | 30 | 2,4x10⁹ |

In a test the case in fraction was extracted with chloroform-methanol and the adherence inhibition was tested on S. pneumoniae and H. influenzae prior to and after extraction. As evident from the Table below the casein fraction was not influenced by the extraction treatment, and thus this denaturation will not influence the adherence inhibition properties.

**TABLE 7**

| % inhibition on | | |
|---|---|---|
| | S. pneumoniae | H. influenzae |
| Prior to extraction | 90 | 92 |
| After extraction | 100 | 86 |
| I.e. no change in the adherence inhibition was seen after extraction compared with prior to. | | |

### COMMENTS

S. pneumoniae and H. influenzae are important causes of morbidity and mortality in all age groups. Respiratory tract infections, e.g., meningitis, otitis, and sinusitis are caused by bacteria which enter via the nasopharynx. Colonization at that site may thus be an important determinant of disease (1). The finding that human casein inhibit attachment of both species opens the possibility to prevent colonization by specific interference with attachment using these structures. The further finding that the casein fraction possesses a bacteriocidal effect against at least S. pneumoniae, separate from the adhesion inhibiting effect, gives a further dimension to this invention.

As evident from the data shown the casein fraction of human milk inhibits the attachment of S. pneumoniae and H. influenzae to human respiratory tract epithelial cells in vitro. Thus it has been demonstrated that the casein fraction inhibits adhesion, although this will not exclude per se that active epithopes on the casein are effective.

The ability of human milk to inhibit attachment has been shown in several systems. Milk contains antibodies to bacterial adhesins, e.g., fimbriae (3, 23). The non-immunoglobulin fraction inhibited the hemagglutination of V. cholerae and E. coli, the attachment to guinea pig intestinal tract and protected against enterotoxin-induced diarrhoea in rabbits (4, 11, 21). Although this activity has been attributed to glycoproteins and free oligosaccharides the exact structures have not been defined, so far. In contrast the milk oligosaccharides which inhibited pneumococcal attachment were identified as specific free oligosaccharides of the lactoseries (3). The inhibitory activity of the milk oligosaccharides was paralleled by synthetic analogues and bacteria agglutinated latex beads covalently coupled with the synthetic oligosaccharide receptors (2, 3).

Unlike for S. pneumoniae, the present casein fraction seemed to contain all the anti-adhesive activity of milk for H. influenzae, none remained in the low molecular weight fraction or in the whey fraction. Thus the oligosaccharide sequences occurring in the free form in the milk did not appear to function as receptors. Like S. pneumoniae, however, the inhibitory activity occurred in human milk. Further studies with the aim to identify the receptor structures for H. influenzae can be based on this species difference.

Depending on storage conditions and other factors the casein fraction of the milk used may undergo detrimental changes with regard to inhibition of adhesion. Thus a inhibition of adhesion of 40 % or more, preferably 50% or more, more preferably 65% or more compared with a saline control may be regarded as an adequate inhibition of adhesion.

### APPLICATIONS

The casein of human origin, can be administered in the form of an oral mucosal dosage unit, an injectable composition, or a topical composition. In any case the casein is normally administered together with commonly known fillers and/or expedients, which are pharmaceutically acceptable.

In case the casein is administered in the form of a solution for topical use the solution contains an emulsifying agent for the case in together with an diluent which can be sprayed into the nasopharynx, or can be inhaled in the form of a mist into the upper respiratory airways.

In oral use the casein is normally administered together with a carrier, which may be a solid, semi-solid or liquid diluent or a capsule. These pharmaceutical preparations are a further object of the present invention. Usually the amount of active compound is between 0.1 to 99 % by weight of the preparation, preferably between 0.5 to 20 % by weight in preparations for injection and between 2 and 50 % by weight in preparations for oral administration.

In pharmaceutical preparations containing a casein of the present invention in the form of dosage units for oral administration the compound may be mixed with a solid, pulverulent carrier, as e.g. with lactose, saccharose, sorbitol, mannitol, starch, such as potatoe starch, corn starch, amylopectin, cellulose derivatives or gelatine, as well as with an antifriction agent such as magnesium stearate, calcium stearate, polyethylene glycol waxes or the like, and be pressed into tablets. Multiple-unit-dosage granules can be prepared as well. Tablets and granules of the above cores can be coated with concentrated solutions of sugar, etc. The cores can also be coated with polymers which change the dissolution rate in the gastrointestinal tract, such as anionic polymers having a pkₐ of above 5.5. Such polymers are hydroxypropylmethyl cellulose phtalate, cellulose acetate phtalate, and polymers sold under the trade mark Eudragit S100 and L100.

In the preparation of gelatine capsules these can be soft or hard. In the former case the active compound is mixed with an oil, and the latter case the multiple-unit-dosage granules are filled therein.

Liquid preparations for oral administration can be present in the form of syrups or suspensions, e.g., solutions containing from about 0.2 % by weight to about 20 % by weight of the active compound disclosed, and glycerol and propylene glycol. If desired, such preparations can contain colouring agents, flavouring agents, saccharine, and carboxymethyl cellulose as a thickening agent.

The daily dose of the active compound varies and is dependent on the type of administrative route, but as a general rule it is 1 to 100 mg/dose of active compound at peroral administration, and 2 to 200 mg/dose in topical administration. The number of applications per 24 hrs depend of the administration route, but may vary, e.g. in the case of a topical application in the nose from 3 to 8 times per 24 hrs, i.a., depending on the flow of phlegm produced by the body treated in therapeutic use. In prophylactic use the number may be on the lower side of the range given.

The topical form can preferably be used in prophylactic treatment, preferably in connection with an infection caused by a rhinitis virus.

The casein can also be used as an additive in infant food, particularly for prophylactic reasons, in order to supply the casein in an easy way to the child. Infants normally reject pharmaceuticals for different reasons. The food product can thus be in the form of a pulverulent porridge base, gruel base, milk substitute base, or more complex food product as of the Scotch collops type, comprising vegetables and meat pieces, often in disintegrated form.

In the case of casein administration to animals they are normally added to the feedstuffs, which besides the caseins contains commonly used nutrients.

In accordance with a further aspect of the invention there is provided a process for determining the presence of S. pneumococci and H. influenzae in a sample taken from the respiratory tract of an animal or human. This process is based on the technique of determining the degree of interaction between the bacteria of the sample and a composition of the present invention. Such interaction may be determined by inhibition or induction of the adherence of the bacteria to cells or other surfaces.

### REFERENCES

1. Andersson, B., Eriksson, B., Falsén, E., Fogh, A., Hanson, L.Å., Nylén, O., Peterson, H., Svanborg Edén, C.,
   Infect. Immun., 32:311-317 (1981).
2. Andersson, B., Dahmén, J., Frejd, T., Leffler, H., Magnusson, G., Noori, G., Svanborg Edén, C.,
   J. Exp. Med. 158:559-570 (1983).
3. Andersson, B., Porres, O., Hanson, L.Å., Lagergård, T., Svanborg Edén, C.,
   J. Infect. Dis. 153:232-237 (1986).
4. Ashkenazi, S., Mirelman, D.,
   Pedriatric Research, 22:130-134 (1987).
5. Branefors-Helander, P.,
   Acta Pathologica, Microbiologica et Immunologica Scandinavia (B), 80:211-220 (1972)
6. Fiat, A.-M., Jollés, J., Aubert, J.-P., Loucheux-Lefebvre, M.-H., Jollés, P.,
   Eur. J. Biochim. 111:333-339 (1980).
7. van Halbeck, H., Dorland, L., Vliegenhart, J.F.G., Fiat, A.-M., Jollés, P.,
   Biochimica et Biophysica Acta. 623:295-300 (1980)
8. van Halbeck, H., Vliegenhart, J.F.G., Fiat, A.-M., Jollés, P.,
   FEBS 187:81-88 (1985).
9. van Heyningen, W.E., Carpenter, C.C.J., Pierce, N.F., Greenough, W.B.,
   J. Infect. Dis. 124:415-418 (1971).
10. van Heyningen, W.E.,
   Nature, 249:415-417 (1974)
11. Holmgren, J., Svennerholm, A.-M., Lindband, M.,
   Infect. Immun. 39:147-154 (1983).
12. Jenness, R.,
   Seminars in Perinatology, 3:225-229 (1979).
13. Kobata, A., In: Horowitz MI and Pigman W (eds), The Glycoconjugates, Academic Press, New York, pp. 423-440 (1977).
14. Källenius, G., Möllby, R., Svensson, S.B., et al,
   FEMS Microbiological Letter, 7:297-302 (1980).
15. Lacks, S., Hotchkiss, R.D.,
   Biochim. Biophys. Acta, 38:508-517 (1060).
16. Leffler, H., Svanborg-Edén, C.,
   FEMS Microbiol. Letter 8:127-134 (1981).
17. Leffler, H., Svanborg Edén, C., In: Mirelman D (ed):
   Microbial Lectins, New York, John Wiley and Sons, pp. 84-96, (1986).
18. Levinthal, W.,
   Zeitschift für Hygiene und Infektionskrankheiten, 76:1-24 (1918).
19. Mellander, L.,
   Upsala Läkarefören. Förhandl. N.F. Bd LII, Häft. 3-4, (1947).
20. Mirelman Ed. L., John Wiley and Sons, Microbial Lectins and Agglutinins; properties and biological activities (1986).
21. Otnaess, A.B., Svennerholm, A.-M.,
   Infect. Immun. 35:738-740 (1982).
22. Porras, O., Svanborg Edén, C., Lagergård, T., Hanson, L.Å.,
   Eur. J. Clin. Microbiol. 4:310-315 (1985).
23. Svanborg Edén, C., Carlsson, B., Hanson, L.Å., Jann,
   B.,Lancet ii:1245 (1979)

## Claims

1. Use of a casein fraction where the active substance is not alfa or beta casein, derived from human milk and having a lower molecular weight limit of 5000 dalton for the preparation of a substrate for the therapeutic, prophylactic, and/or diagnostic use in infections of the respiratory tract caused by S. pneumoniae and/or H. influenzae.

2. Method for diagnosing infections caused by S. pneumoniae and/or H. influenzae, wherein a sample extracted from the infected mammals, including man is determined with regard to adhesion vis-à-vis a composition of the claim 1.

## Patentansprüche

1. Verwendung einer Caseinfraktion, wobei die aktive Substanz nicht Alpha- oder Betacasein ist, abstammend von menschlicher Milch und aufweisend eine untere Molekulargewichtsgrenze von 5.000 Dalton, zur Herstellung eines Substrates für die therapeutische, prophylaktische und/oder diagnostische Verwendung bei Infektionen der Atemwege, die durch S. Pneumoniae und/oder H. Influenzae hervorgerufen sind.

2. Verfahren zur Diagnose von durch S. Pneumoniae und/oder H. Influenzae hervorgerufenen Infektionen, wobei eine aus den infizierten Säugern, einschließlich des Menschen, entnommene Probe hinsichtlich eines Anhaftens an eine Zusammensetzung gemäß Anpruch 1 bestimmt wird.

## Revendications

1. Utilisation d'une fraction de caséine dans laquelle la substance active n'est pas la caséine alpha ou bêta, dérivée du lait humain et possédant une limite inférieure de poids moléculaire de 5000 daltons pour la préparation d'un substrat destiné a une utilisation thérapeutique, prophylactique et/ou diagnostique dans les infections des voies respiratoires provoquées par S. pneumoniae et/ou par H. influenzae.

2. Procédé de diagnostic des infections provoquées par S. pneumoniae et/ou par H. influenzae, dans lequel un échantillon prélevé chez les mammifères infectés, y compris chez l'homme, est dosé afin de déterminer l'adhésion vis-à-vis d'une composition selon la revendication 1.
